# EUROPEAN PATENT SPECIFICATION

(11) **EP 4 186 537 B1**
(45) Date of publication and mention of the grant of the patent: **20.08.2025**
(21) Application number: 20945919.7
(22) Date of filing: 28.08.2020
(51) Int. Cl.: C08J 3/075, C08L 71/02, C08L 67/00, C08L 5/08, A61L 24/00, A61L 24/04

(54) **SEALING HYDROGEL, PREPARATION METHOD THEREFOR AND APPLICATION THEREOF**
DICHTUNGSHYDROGEL, HERSTELLUNGSVERFAHREN DAFÜR UND ANWENDUNG DAVON
HYDROGEL DE SCELLEMENT, SA MÉTHODE DE PRÉPARATION ET SON APPLICATION

(30) Priority: 23.07.2020 CN 202010718193
(43) Date of publication of application: 31.05.2023
(73) Proprietor: NKD pharm Co., Ltd, Beijing 100176 (CN)
(72) Inventor: LENG, Hongfei, Beijing 100176 (CN); XU, Xiaoyu, Beijing 100176 (CN); TAO, Xiumei, Beijing 100176 (CN)
(74) Representative: Wichmann, Hendrik
(86) International application number: PCT/CN2020/112194
(87) International publication number: WO 2022/016664

(56) References cited:
- WO-A1-2014/157186
- CN-A- 105 169 469
- CN-A- 105 778 124
- CN-A- 106 913 902
- CN-A- 110 433 344
- CN-A- 110 643 057
- KR-A- 20020 089 772
- US-A1- 2020 206 367
- JOHNSTON TREVOR G., FELLIN CHRISTOPHER R., CARIGNANO ALBERTO, NELSON ALSHAKIM: "Poly(alkyl glycidyl ether) hydrogels for harnessing the bioactivity of engineered microbes", FARADAY DISCUSSIONS, ROYAL SOCIETY OF CHEMISTRY, CAMBRIDGE, GB, vol. 219, 30 October 2019 (2019-10-30), GB , pages 58 - 72, XP055889210, ISSN: 1359-6640, DOI: 10.1039/C9FD00019D

## Description

### Technical Field

The present invention relates to the technical field of polymer materials, and specifically, to a sealing hydrogel, and a preparation method therefor.

### Background Art

Hydrogel products developed based on PEG derivatives have the advantages of good biocompatibility, non-toxicity, non-irritation, biodegradability, no need to be removed again after surgery in clinical application, and the products will gradually degrade with the recovery of patients and be metabolized by kidneys. At present, there are many kinds of hydrogel sealants developed based on PEG on the market, which are used for wound sealing, anti-adhesion, leakage sealing and the like. In recent years, the incidence of cerebrospinal fluid leakage (abbreviated as CSFL) has tended to increase with the increase in patients with spinal revision surgery and severe spinal dura mater adhesions and advanced age. Proper treatment of cerebrospinal fluid leakage is of great significance to achieve successful spinal surgery and prevent serious complications. In clinical use, because of the risk of nerve compression, the swelling ratio of the sealing hydrogel for spinal wound, spinal dura mater or cerebral dura mater should be as low as possible. In addition, for patients with other treatment needs such as inflammation, it is necessary not only to seal the wound but also to load drugs for treatment. In addition, the low-swelling PEG hydrogel used in the current technology has strong acidity and alkalinity, and there is adverse irritation to tissues during use. The currently available sealing hydrogel has a large swelling ratio and cannot be loaded with drugs or its sealing performance will be affected after loading drugs. Therefore, there is an urgent need for a drug-loaded hydrogel with low swelling for the sealing of the spine, cerebral dura mater and the like. WO 2014/157186 A1 discloses a hydrogel composition which comprises, as constituents, both a tetra-armed polymer having a polyethylene glycol skeleton and a temperature-responsive tetra-armed polymer having a phase transition temperature and in which the polymers are crosslinked at the terminals through covalent bonds to form a network structure. CN 105 169 469 A discloses a tissue sealant and a preparation method and application thereof, wherein the sealant mainly comprises hydrophilic raw material, nucleophilic raw material, a marker and physiological thinner, wherein the hydrophilic raw material mainly refers to the derivatives of polyethylene glycol and the nucleophilic raw material mainly refers to polyamino acid.

### Summary of the Invention

Directing at the problems existing in the prior art, the present invention provides a sealing hydrogel, and precursors for preparing the sealing hydrogel includes a first component and a second component.

The first component includes a compound A and a compound B; the compound A is a multiarm polyethylene glycol derivative, and the compound B is a polyarm copolyester of glycidyl isopropyl ether and ethyl glycidyl ether.

The second component includes an oligopeptide .

Preferably, both the compound A and the compound B contain the following group:

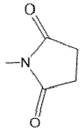

Preferably, the compound A has a general structural formula of: 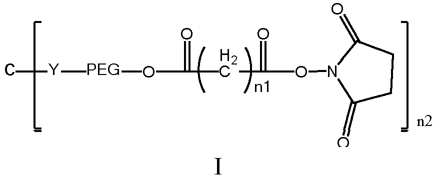
wherein n1 is an integer from 2 to 8 and n2 is an integer from 4 to 8; the molecular weight of PEG is 1250 to 8000, and the PDI of PEG ≤ 1.02;
Y is a same or a different linking group that is one or more selected from -O-, - O(CH₂CH₂)ᵢ-, -(CH₂CH₂)ᵢ-, -(CH₂)ᵢCONH-, -OOC(CH₂)ᵢCOO-, -OOCNH(CH₂)ᵢNHCOO- and -OOC(CH₂)ᵢCONH-; and i is an integer from 0 to 20;
the compound B has a general structural formula of: 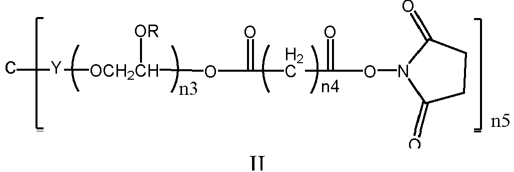
wherein, n3 is an integer from 11 to 70, n4 is an integer from 2 to 8, and n5 is an integer from 4 to 8;
the groups that Y in general formula II can be selected from are the same as the groups that Y in general formula I can be selected from; and R is ethyl or isopropyl.

Preferably, the sealing hydrogel has a swelling ratio in the three-dimensional direction greater than or equal to -5% and less than or equal to 5%.

Preferably, in the compound B, in per 100 parts of ethyl glycidyl ether and glycidyl isopropyl ether, there are 60 to 80 parts of ethyl glycidyl ether included.

Preferably, the compound A and compound B have the same number of arms.

Preferably, the compound A and compound B have the same molecular weight.

Preferably, in per 100 parts of the mixture of compound A and compound B, there are 40 to 60 parts of compound A included.

Preferably, a molar ratio of the functional group 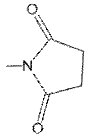 in the compound A and compound B to the functional group -NH₂ in the oligopeptide is 1:(1 to 2).

Preferably, the first component further contains a color developer;
further preferably, the ratio of the color developer to the total mass of compound A and compound B is (0.0001 to 0.01):1;
more further preferably, the color developer is fluorescein.

Preferably, the number of residues of amino acids in the oligopeptide is from 2 to 6; and preferably, the oligopeptide is trilysine.

Preferably, the second component further includes a bacteriostatic agent;
further preferably, the bacteriostatic agent is a chitosan derivative,
more further preferably, the chitosan derivative is carboxymethyl chitosan, and the mass ratio of the bacteriostatic agent to the oligopeptide is (0.015 to 0.1):1.

Preferably, the first component and/or the second component is added with a drug;
further preferably, the drug is at least one of an anti-inflammatory drug, a hemostatic drug, an analgesic drug, an anti-rejection drug and an anti-tumor drug.

Preferably, the sealing hydrogel is prepared by the following method:
(1) mixing the first component and the second component with buffer a to obtain a mixed solution system; and
(2) mixing buffer b with the mixed solution system to obtain a sealing hydrogel;
preferably, the buffer a has a pH of 6.0 to 7.5; and/or, the buffer b has a pH of 9.0 to 9.5.

Preferably, in the mixed solution system, the oligopeptide has a concentration of 1 to 7 mg/mL;
preferably, in the mixed solution system, the total concentration of compound A and compound B is 40 to 100 mg/mL, preferably 40 to 75 mg/mL. Preferably, the buffer a contains one or more of phosphate, sodium chloride, potassium chloride, phosphoric acid and hydrochloric acid; and/or,
the buffer b contains one or more of phosphate, carbonate, borate and sodium hydroxide.

Preferably, the volume ratio of buffer a to the buffer b is 1:(0.8 to 1.2).

The present invention also provides a method for preparing the sealing hydrogel according to the present invention, comprising the following steps:
(1) mixing the first component and the second component with buffer a to obtain a mixed solution system; and
(2) mixing buffer b with the mixed solution system to obtain a sealing hydrogel; preferably, the buffer a has a pH of 6.0 to 7.5; and/or, the buffer b has a pH of 9.0 to 9.5.

The present invention also provides a kit for preparing the sealing hydrogel according to the present invention, wherein a duplex syringe is arranged in the kit, the first component and second component are dissolved in buffer a and stored in syringe 1; and the buffer b is stored in syringe 2.

The present invention also protects the sealing hydrogel according to the present invention for use in methods of sealing of spinal dura mater and cerebral dura mater, leak sealing of other tissues, wound sealing and anti-adhesion.

The present invention has the following beneficial effects:
(1) in the present invention, the swelling ratio of the hydrogel can be effectively reduced by selecting the high molecular weight compound in the gel component, and mixing the first component with the second component.
(2) The hydrogel described in the present invention still has high sealing strength at low polymer concentration and after drug loading, therefore the degradation products may be reduced, the impact of metabolites *in vivo* on human body may be reduced, the cost of raw materials may be reduced, and at the same time, drugs can be loaded for treatment.
(3) The hydrogel described in the present invention has good drug release performance after drug loading, and the release rate is moderate, which is conducive to effective treatment of diseases.
(4) The hydrogel described in the present invention has less acidic and alkaline buffer, which is closer to the neutral human environment, with less irritation and low cytotoxicity;
(5) The hydrogel described in the present invention has antibacterial properties, which can reduce the use of antibiotics.
(6) The hydrogel described in the present invention has a color developer fluorescein, which enables tracer observation of hydrogel for *in vivo* application.

### Specific Modes for Carrying Out the Embodiments

The present application will be described in detail by the following additional technical features.

The present invention provides a sealing hydrogel, and precursors for preparing the sealing hydrogel includes a first component and a second component;
the first component includes compound A and compound B; the compound A is a multiarm polyethylene glycol derivative, and the compound B is a polyarm copolyester of glycidyl isopropyl ether and ethyl glycidyl ether;
the second component includes an oligopeptide.

The sealing hydrogel described in the present application, by using compound B in combination with compound A, and then further combined with the second component, can significantly reduce the swelling ratio, and the obtained hydrogel has weak acidity and alkalinity, and there is no adverse stimulation to tissues during use compared with the hydrogel prepared by using compound A and the second component alone in the prior art. Moreover, this hydrogel can be loaded with drugs, and the sealing property of hydrogel will not be affected after drug loading.

According to some preferred examples, both the compound A and the compound B contain the following group:

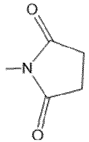

According to some preferred examples, the compound A has a general structural formula of: 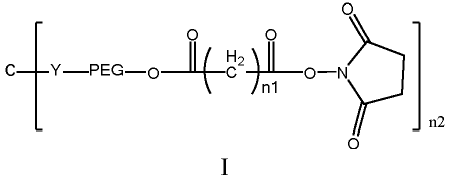
wherein, n1 is an integer from 2 to 8 and n2 is an integer from 4 to 8; the molecular weight of PEG is 1250 to 8000, and the PDI of PEG ≤ 1.02;
Y is a same or a different linking group that is one or more selected from -O-, - O(CH₂CH₂)ᵢ-, -(CH₂CH₂)ᵢ-, -(CH₂)ᵢCONH-, -OOC(CH₂)ᵢCOO-, -OOCNH(CH₂)ᵢNHCOO- and -OOC(CH₂)ᵢCONH-; and i is an integer from 0 to 20;
the compound B has a general structural formula of: 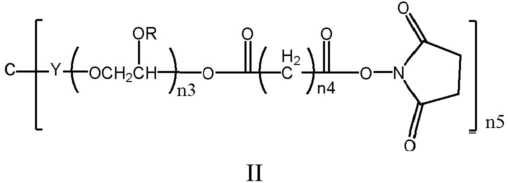
wherein, n3 is an integer from 11 to 70, n4 is an integer from 2 to 8, and n5 is an integer from 4 to 8;
the groups that Y in general formula II can be selected from are the same as the groups that Y in general formula I can be selected from; and R is ethyl or isopropyl.

According to some preferred examples, the sealing hydrogel has a swelling ratio in the three-dimensional direction greater than or equal to -5% and less than or equal to 5%.

Because the above raw materials are selected, the hydrogel of the present invention can be prepared with a swelling ratio of more than or equal to -5% and less than or equal to 5%, which can meet the requirements of clinical treatment with the risk of nerve compression, can be applied to the sealing of spinal wounds, spinal dura mater or cerebral dura mater, and has the advantages of high safety and good effect.

According to some preferred examples, in the compound B, in per 100 parts of ethyl glycidyl ether and glycidyl isopropyl ether, there are 60 to 80 parts of ethyl glycidyl ether ethyl glycidyl ether comprised, and the groups in the compound B are within the above range, so that the gel obtained has a small swelling ratio.

According to some preferred examples, the compound A and compound B have the same number of arms. Under the above conditions, the gel obtained has a small swelling ratio.

According to some preferred examples, the compound A and compound B have the same molecular weights. Under the above conditions, the gel obtained has a small swelling ratio.

According to some preferred examples, in per 100 parts of the mixture of compound A and compound B, there are 40 to 60 parts of compound A comprised. Under the above conditions, the gel obtained has a small swelling ratio.

According to some preferred examples, a molar ratio of the functional group 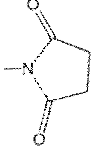 in the compound A and compound B to the functional group -NH₂ in the oligopeptide is 1:(1 to 2). Under the above conditions, the gel obtained has a small swelling ratio.

Under the condition that the hydrogel of the present invention meets the above conditions, it is beneficial to further reduce the swelling ratio of the gel, and the swelling ratio in the three-dimensional direction can even reach about 2% to 3%.

According to some preferred examples, the compound A is one or more selected from 4-arm polyethylene glycol succinimide succinate (molecular weight 5000 to 32000 Da), 4-arm-polyethylene glycol succinimide glutarate (molecular weight 5000 to 32000 Da), 4-arm-polyethylene glycol succinimide adipate (molecular weight 5000 to 32000 Da), 4-arm-polyethylene glycol succinimide heptanedioate (molecular weight 5000 to 32000 Da), 4-arm-polyethylene glycol succinimide sebacate (molecular weight 5000 to 32000 Da), 6-arm-polyethylene glycol succinimide succinate (molecular weight 5000 to 32000 Da), 6-arm-polyethylene glycol succinimide glutarate (molecular weight 5000 to 32000 Da), 6-arm-polyethylene glycol succinimide adipate (molecular weight 5000 to 32000 Da) 6-arm-polyethylene glycol succinimide heptanedioate (molecular weight 5000 to 32000 Da), 6-arm-polyethylene glycol succinimide sebacate (molecular weight 5000 to 32000 Da), 8-arm-polyethylene glycol succinimide succinate (molecular weight 5000 to 32000 Da), 8-arm-polyethylene glycol succinimide glutarate (molecular weight 5000 to 32000 Da), 8-arm-polyethylene glycol succinimide adipate (molecular weight 5000 to 32000 Da), 8-arm-polyethylene glycol succinimide heptanedioate (molecular weight 5000 to 32000 Da) and 8-arm-polyethylene glycol succinimide sebacate (molecular weight 5000 to 32000 Da).

According to some preferred examples, the compound B is selected from 4-arm-copolyether succinimide succinate (molecular weight 5000 to 32000 Da), 4-arm-copolyether succinimide glutarate (molecular weight 5000 to 32000 Da), 4-arm-copolyether succinimide adipate (molecular weight 5000 to 32000 Da), 4-arm-copolyether succinimide heptanedioate (molecular weight 5000 to 32000 Da), 4-arm-copolyether succinimide sebacate (molecular weight 5000 to 32000 Da), 6-arm-copolyether succinimide succinate (molecular weight 5000 to 32000 Da), 6-arm-copolyether succinimide glutarate (molecular weight 5000 to 32000 Da), 6-arm-copolyether succinimide adipate (molecular weight 5000 to 32000 Da), 6-arm-copolyether succinimide heptanedioate (molecular weight 5000 to 32000 Da), 6-arm-copolyether succinimide sebacate (molecular weight 5000 to 32000 Da), 8-arm-copolyether succinimide succinate (molecular weight 5000 to 32000 Da), 8-arm-copolyether succinimide glutarate (molecular weight 5000 to 32000 Da), 8-arm-copolyether succinimide adipate (molecular weight 5000 to 32000 Da), 8-arm-copolyether succinimide heptanedioate (molecular weight 5000 to 32000 Da), and 8-arm-copolyether succinimide sebacate (molecular weight 5000 to 32000 Da);

According to some preferred examples, the compound A is 4-arm-polyethylene glycol succinimide adipate or 8-arm- polyethylene glycol succinimide succinate, and the compound B is 4-arm-copolyether succinimide adipate, 8-arm-copolyether succinimide succinate or 4-arm-copolyether succinimide glutarate.

According to some preferred examples, the first component also contains a developer;
further preferably, the ratio of the color developer to the total mass of compound A and compound B in the first component is (0.0001 to 0.01): 1;
more further preferably, the color developer is fluorescein. The first compound of the present invention can bind well to the fluorescein, and thus a hydrogel with fluorescent properties can be prepared.

According to some preferred examples, the number of amino acid residues in the oligopeptide is from 2 to 6, and the above selection can ensure that the prepared gel has a small swelling ratio and a fast gelation time.

Further preferably, the oligopeptide is trilysine.

According to some preferred examples, the second component further includes a bacteriostatic agent; and the second component of the present invention can ideally bind with the bacteriostatic agent, so that the obtained gel has certain bacteriostatic effect.

Further preferably, the bacteriostatic agent is a chitosan derivative. The bacteriostatic agent above is both antibacterial and safe for *in vivo* implantation.

More further preferably, the chitosan derivative is carboxymethyl chitosan, with the mass ratio of (0.015 to 0.1):1 to the oligopeptide.

According to the present invention, the color developer is added into the first component, and the bacteriostatic agent is added into the second component, so that the gel reaction is not affected, and the gel formation is facilitated.

According to some preferred examples, drugs are added to the first component and/or the second component.

According to some preferred examples, the drug is at least one of an anti-inflammatory drug, a hemostatic drug, an analgesic drug, an anti-rejection drug and an anti-tumor drug.

According to some preferred examples, the oligopeptide of the present invention is prepared by a method including the following steps:
(1) mixing the first component and the second component with buffer a to obtain a mixed solution system; and
(2) mixing buffer b with the mixed solution system to obtain a sealing hydrogel; in order to prepare a sealing hydrogel with good effect, the method described above for preparing hydrogel in the present invention is further optimized on the basis of the selected raw materials, and the performance of the final hydrogel can be effectively improved by adopting the above steps for preparing the hydrogel.

According to some preferred examples, the pH of the buffer a is 6.0 to 7.5, and the pH of the buffer b is 9.0 to 9.5. When the pH of the buffer is in the above range, not only the forming characteristics of hydrogel is ensured, but also no damage to tissue cells is caused.

According to some preferred examples, in the mixed solution system, the oligopeptide has a concentration of 1 to 7 mg/ml;
According to some preferred examples, in the mixed solution system, the total concentration of compound A and compound B is 40 to 100 mg/mL, preferably 40 to 75 mg/mL.

When the effective components in the first component and the second component are within the above range, the swelling ratio and sealing strength of the gel can be ensured to be in the desired range.

According to some preferred examples, the volume ratio of the buffer a to the buffer b is 1: (0.8 to 1.2).

According to some preferred examples, the buffer a contains one or more of phosphate, sodium chloride, potassium chloride, phosphoric acid and hydrochloric acid;
According to some preferred examples, the buffer b contains one or more of phosphate, carbonate, borate and sodium hydroxide.

The present invention also protects the method for preparing the hydrogel of the present invention, which comprises the following steps:
(1) mixing the first component and the second component with buffer a to obtain a mixed solution system; and
(2) mixing buffer b with the mixed solution system to obtain a sealing hydrogel.

The present invention also protects a kit for preparing the sealing hydrogel of the present invention, wherein a duplex syringe is arranged in the kit, and the first component and the second component are dissolved in buffer a and stored in syringe 1; and the buffer solution b is stored in syringe 2.

According to some preferred examples, during the use of the kit according to the present invention, drugs can be added to solution a or solution b, drugs that are acidic or neutral are added to solution a, and drugs that are stable in alkaline conditions are added to solution b.

The present invention also protects the sealing hydrogel for use in the sealing of spinal dura mater and cerebral dura mater, leak sealing of other tissues, wound sealing and anti-adhesion.

The present application will be further described in detail by the following examples, see Table 1 for details:

**Table 1**

| Examples | The first component | | | The second component | | | | |
|---|---|---|---|---|---|---|---|---|
| | PEG derivative, mg | Copolyether polyarm copolyester, mg | Fluorescein 1, mg | Trilysine acetate, mg | Carboxym ethyl chitosan, mg | Molar ratio of functional groups of the first component to the second component | Buffer a | Buffer b |
| 1 | 4-arm polyethylene glycol succinimide adipate, molecular weight 10,000, 20 mg | 4-arm-copolyether succinimide adipate, molecular weight 10,000, 20 mg, with a molar ratio of ethyl glycidyl ether to glycidyl isopropyl ether of 60:40 | 0.004 mg | 1.6 | 0.024 | 1:1 | Phosphate buffer, pH=7.5, 1ml | Borax buffer, pH=9.0, 1ml |
| 2 | 4-arm polyethylene glycol succinimide adipate, molecular weight 10,000, 16 mg | 4-arm-copolyether succinimide adipate, molecular weight 10,000, 24 mg, with a molar ratio of ethyl glycidyl ether to glycidyl isopropyl ether of 80:20 | 0.4 mg | 1.6 | 0.024 | 1:1 | Phosphate buffer, pH=7.5, 1ml | Borax buffer, pH=9.0, 1ml |
| 3 | 4-arm polyethylene glycol succinimide adipate, molecular weight 10,000, 24 mg | 4-arm-copolyether succinimide adipate, molecular weight 10,000, 16 mg, with a molar ratio of ethyl glycidyl ether to glycidyl isopropyl ether of 70:30 | 0.4 mg | 1.6 | 0.024 | 1:1 | Phosphate buffer, pH=7.5, 1ml | Borax buffer, pH=9.0, 1ml |
| 4 | 4-arm polyethylene glycol succinimide adipate, molecular weight 32,000, 24 mg | 4-arm-copolyether succinimide adipate, molecular weight 32,000, 16 mg, with a molar ratio of ethyl glycidyl ether to glycidyl isopropyl ether of 60:40 | 0.4 mg | 1.0 | 0.024 | 1:2 | Phosphate buffer, pH=7.5, 1ml | Borax buffer, pH=9.0, 1ml |
| 5 | 4-arm polyethylene glycol succinimide adipate, molecular weight 10,000, 50 mg | 4-arm-copolyether succinimide adipate, molecular weight 10,000, 50 mg, with a molar ratio of ethyl glycidyl ether to glycidyl isopropyl ether of 60:40 | 0.01 mg | 4.0 | 0.096 | 1:1 | Phosphate buffer, pH=7.5, 1ml | Borax buffer, pH=9.0, 1ml |
| 6 | 4-arm polyethylene glycol succinimide adipate, molecular weight 10,000, 37.5 mg | 4-arm-copolyether succinimide adipate, molecular weight 10,000, 37.5 mg, with a molar ratio of ethyl glycidyl ether to glycidyl isopropyl ether of 65:35 | 0.0075 mg | 3.0 | 0.072 | 1:1 | Phosphate buffer, pH=6, 1ml | Borax buffer, pH=9.5, 1ml |
| 7 | 8-arm-polyethylene glycol succinimide succinate, molecular weight 10,000, 43.7 mg | 8-arm-copolyether succinimide succinate, molecular weight 10,000, 43.7 mg, with a molar ratio of ethyl glycidyl ether to glycidyl isopropyl ether of 75:35 | 0.087 mg | 7.0 | 0.035 | 1:1 | Phosphate buffer, pH=7.5, 1ml | Borax buffer, pH=9.0, 1ml |
| 8 | 4-arm polyethylene glycol succinimide adipate, molecular weight 10,000, 30 mg | 4-arm-copolyether succinimide adipate, molecular weight 10,000, 30 mg, with a molar ratio of ethyl glycidyl ether to glycidyl isopropyl ether of 60:40 | 0.006 mg | 3.6 | 0.036 | 1:1.5 | Phosphate buffer, pH=6.5, 1ml | Borax buffer, pH=9.0, 1ml |
| 9 | 4-arm polyethylene glycol succinimide adipate, molecular weight 10,000, 30 mg | 4-arm-copolyether succinimide adipate, molecular weight 10,000, 30 mg, with a molar ratio of ethyl glycidyl ether to glycidyl isopropyl ether of 60:40 | 0.006 mg | 3.6 | 0.036 | 1:1.5 | Phosphate buffer, pH=6, 1ml | Borax buffer, pH=9.0, 1ml |
| 10 | 4-arm polyethylene glycol succinimide glutarate, molecular weight 20,000, 30 mg | 4-arm-copolyether succinimide glutarate, molecular weight 20,000, 30 mg, with a molar ratio of ethyl glycidyl ether to glycidyl isopropyl ether of 60:40 | 0.006 mg | 1.8 | 0.018 | 1:1.5 | Phosphate buffer, pH=6, 1ml | Borax buffer, pH=9.0, 1ml |
| 11 | 4-arm polyethylene glycol succinimide adipate, molecular weight 10,000, 20 mg | 4-arm-copolyether succinimide adipate, molecular weight 20,000, 20 mg, with a molar ratio of ethyl glycidyl ether to glycidyl isopropyl ether of 60:40 | 0.004 mg | 1.6 | 0.024 | 1:1.3 | Phosphate buffer, pH=6.5, 1ml | Borax buffer, pH=9.0, 1ml |
| 12 | 4-arm polyethylene glycol succinimide adipate, molecular weight 10,000, 20 mg | 6-arm-copolyether succinimide adipate, molecular weight 10,000, 20 mg, with a molar ratio of ethyl glycidyl ether to glycidyl isopropyl ether of 60:40 | 0.4 mg | 1.6 | 0.024 | 1:0.8 | Phosphate buffer, pH=6.5, 1ml | Borax buffer, pH=9.0, 1ml |
| 13 | 4-arm polyethylene glycol succinimide adipate, molecular weight 10,000, 20 mg | 4-arm-copolyether succinimide adipate, molecular weight 10,000, 20 mg, with a molar ratio of ethyl glycidyl ether to glycidyl isopropyl ether of 50:50 | 0.004 mg | 1.6 | 0.024 | 1:1 | Phosphate buffer, pH=7.5, 1ml | Borax buffer, pH=9.0, 1ml |
| 14 | 4-arm polyethylene glycol succinimide adipate, molecular weight 10,000, 12 mg | 4-arm-copolyether succinimide adipate, molecular weight 10,000, 28 mg, with a molar ratio of ethyl glycidyl ether to glycidyl isopropyl ether of 60:40 | 0.004 mg | 1.6 | 0.024 | 1:1 | Phosphate buffer, pH=7.5, 1ml | Borax buffer, pH=9.0, 1ml |
| 15 | 4-arm polyethylene glycol succinimide adipate, molecular weight 10,000, 20 mg | 4-arm-copolyether succinimide adipate, molecular weight 10,000, 20 mg, with a molar ratio of ethyl glycidyl ether to glycidyl isopropyl ether of 60:40 | 0.004 mg | 1.2 | 0.018 | 1:0.75 | Phosphate buffer, pH=7.5, 1ml | Borax buffer, pH=9.0, 1ml |
| 16 | 4-arm polyethylene glycol succinimide adipate, molecular weight 10,000, 55 mg | 4-arm-copolyether succinimide adipate, molecular weight 10,000, 55 mg | 0.011 mg | 6.6 | 0.066 | 1:1.5 | Phosphate buffer, pH=6.5, 1ml | Borax buffer, pH=9.0, 1ml |
| 17 | 4-arm polyethylene glycol succinimide adipate, molecular weight 10,000, 15 mg | 4-arm-copolyether succinimide adipate, molecular weight 10,000, 15 mg | 0.003 mg | 1.8 | 0.018 | 1:1.5 | Phosphate buffer, pH=6.5, 1ml | Borax buffer, pH=9.0, 1ml |
| 18 | 4-arm polyethylene glycol succinimide adipate, molecular weight 10,000, 30 mg | 4-arm-copolyether succinimide adipate, molecular weight 10,000, 30 mg | 0.006 mg | 3.6 | 0.036 | 1:1.5 | Phosphate buffer, pH=6.5, 1ml | Borax buffer, pH=10.0, 1ml |
| Comparati ve Example 1 | 4-arm polyethylene glycol succinimide adipate, molecular weight 10,000, 40 mg | 0 | 0.004 mg | 1.6 | 0.024 | 1:1 | Phosphate buffer, pH=7.5, 1ml | Borax buffer, pH=90, 1ml |

The properties of the hydrogels in the above Examples were characterized, and the results are shown in Table 2:

**Table 2**

| Examples | Swelling ratio, % | | | PH difference of leaching solution | Sealing strength, mmHg | Relative cell proliferation rate, % |
|---|---|---|---|---|---|---|
| | Length | Width | Height | | | |
| 1 | 1.5 | 1.8 | 2.2 | 0.6 | 98 | 87 |
| 2 | -2.5 | -2.3 | -2.6 | 0.9 | 101 | 85 |
| 3 | 2.1 | 1.9 | 2.1 | 0.7 | 97 | 85 |
| 4 | 3.5 | 3.2 | 3.2 | 0.9 | 87 | 81 |
| 5 | 4.6 | 4.2 | 3.9 | 0.2 | 110 | 92 |
| 6 | 2.8 | 2.7 | 2.5 | 0.2 | 96 | 92 |
| 7 | 2.9 | 3.0 | 3.0 | 0.8 | 91 | 86 |
| 8 | 1.0 | 1.2 | 0.5 | 0.3 | 105 | 92 |
| 9 | 1.1 | 1.5 | 0.8 | 0.2 | 95 | 91 |
| 10 | 1.2 | 1.6 | 1.0 | 0.2 | 97 | 91 |
| 11 | 8.6 | 6.3 | 8.7 | 0.5 | 85 | 75 |
| 12 | 5.6 | 5.2 | 6.1 | 0.5 | 86 | 77 |
| 13 | 6.2 | 5.6 | 7.2 | 0.5 | 83 | 76 |
| 14 | 6.1 | 6.1 | 7.5 | 0.4 | 82 | 77 |
| 15 | 7.9 | 7.5 | 7.2 | 0.5 | 83 | 77 |
| 16 | 6.8 | 5.6 | 5.3 | 0.3 | 89 | 85 |
| 17 | 1.5 | 1.6 | 1.2 | 0.8 | 56 | 88 |
| 18 | 1.2 | 1.5 | 0.9 | 1.7 | 102 | 75 |
| Comparative Example 1 | 6.7 | 7.9 | 11.2 | 0.8 | 96 | 83 |

It can be seen from the above Examples and Comparative Example that the present invention can indeed reduce the swelling ratio of the materials by the selection of raw materials for the preparation of hydrogel, especially the selection of compound B. Further, by optimizing the selection of compound B, the relative amounts used of compound A and compound B, the molecular weight and arm number and the ratio of functional groups in the first component and the second component, and the pH of the buffer and other conditions, the swelling ratio can be further reduced, the overall performance can be improved, and a hydrogel with low swelling ratio, high sealing strength and ideal for cell growth can be finally obtained.

The drug loading performance of the above Examples was tested, and the results are shown in Table 3.

Drug loading: the ratio of each component is the same as that in Example 8

**Table 3**

| The first component | | | The second component | | | |
|---|---|---|---|---|---|---|
| PEG derivative, mg | Copolyether polyarm copolyester, mg | Edible brilliant blue 1, mg | Trilysine acetate, mg | Carboxymethyl chitosan, mg | Buffer a | Buffer b |
| 4-arm polyethylene glycol succinimide adipate, molecular weight 10,000, 80mg | 4-arm-copolyether succinimide adipate, molecular weight 10,000, 80 mg | 0.016 mg | 9.6 | 0.096 | Phosphate buffer, pH=6.5 | Borax buffer, pH=9.0 |

### Test results on Drug loading:

| Experiment Example | Drug loading | Buffer a | Buffer b | Swelling ratio, % | | | Sealing strength, mmHg | Release time, d |
|---|---|---|---|---|---|---|---|---|
| | | | | Length | Width | Height | | |
| 1 | Methylprednisolone 40 mg | 4 ml | 4 ml | 1.1 | 1.1 | 0.7 | 103 | 28 |
| 2 | Methylprednisolone 120 mg | 4 ml | 4 ml | 1.3 | 1.2 | 0.5 | 101 | 35 |
| 3 | Diprospan 1 ml | 3 ml | 4 ml | 1.2 | 1.1 | 0.5 | 102 | 41 |
| 4 | Diprospan 2 ml | 2 ml | 4 ml | 1.2 | 1.2 | 0.6 | 101 | 43 |

The loading amount of Methylprednisolone in Experiment Example 1 was the amount of commercially available single bottle, and Experiment Example 2 was to increase the loading amount of Methylprednisolone by 3 times, and then the results were tested, and there was no significant decrease after increasing the loading amount from the swelling ratio rate and sealing strength. Experiment Example 4 is the effect of loading one commercially available Diprospan injection. From the results, there is no significant effect on the performance of the gel itself after loading Diprospan, and the sealing hydrogel described in the present invention has a short drug release period.

Although the present invention has been described in detail by general description and specific embodiments, it is obvious to a person skilled in the art that some modifications or improvements can be made on the basis of the present invention.

### Industrial applicability

The present invention provides a sealing hydrogel, a preparation method therefor and an application thereof. Raw materials for preparing the sealing hydrogel comprise a first component and a second component. The first component comprises a compound A and a compound B. The compound A is a multiarm polyethylene glycol derivative, and the compound B is polyarm copolyester of glycidyl isopropyl ether and ethyl glycidyl ether. The second component comprises oligopeptide. By means of the selection of a high molecular weight compound in gel components and the coordination of the first component and the second component, the swelling ratio of the hydrogel can be effectively reduced, with good economic value and application prospects.

## Claims

1. A sealing hydrogel, wherein precursors for preparing the sealing hydrogel comprise a first component and a second component;
the first component comprises compound A and compound B; the compound A is a multiarm polyethylene glycol derivative, and the compound B is a polyarm copolyester of glycidyl isopropyl ether and ethyl glycidyl ether;
the second component comprises an oligopeptide.

2. The sealing hydrogel according to claim 1, wherein both the compound A and compound B contain the following group:

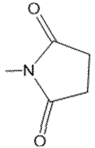

3. The sealing hydrogel according to claim 1 or 2, wherein the compound A has a general structural formula of: 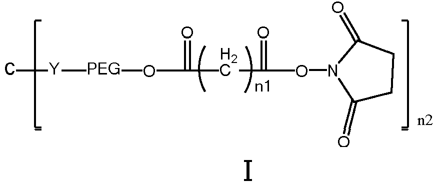
wherein, n1 is an integer from 2 to 8 and n2 is an integer from 4 to 8; the molecular weight of PEG is 1250 to 8000, and the PDI of PEG ≤ 1.02;
Y is a same or a different linking group that is one or more selected from -O-, - O(CH₂CH₂)ᵢ-, -(CH₂CH₂)ᵢ-, -(CH₂)ᵢCONH-, -OOC(CH₂)ᵢCOO-, -OOCNH(CH₂)ᵢNHCOO- and -OOC(CH₂)ᵢCONH-; and i is an integer from 0 to 20;
the compound B has a general structural formula of: 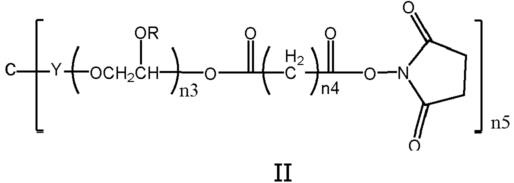
wherein, n3 is an integer from 11 to 70, n4 is an integer from 2 to 8, and n5 is an integer from 4 to 8;
the groups that Y in general formula II can be selected from are the same as the groups that Y in general formula I can be selected from; and R is ethyl or isopropyl.

4. The sealing hydrogel according to any one of claims 1 to 3, wherein the sealing hydrogel has a swelling ratio in the three-dimensional direction greater than or equal to -5% and less than or equal to 5%.

5. The sealing hydrogel according to claim 1, wherein in the compound B, in per 100 parts of ethyl glycidyl ether and glycidyl isopropyl ether, there are 60 to 80 parts of ethyl glycidyl ether comprised.

6. The sealing hydrogel according to claim 1, wherein the compound A and compound B have the same number of arms; or
wherein the compound A and compound B have the same molecular weight; or
wherein in per 100 parts of the mixture of compound A and compound B, there are 40 to 60 parts of compound A comprised.

7. The sealing hydrogel according to any one of claims 2 to 6, wherein a molar ratio of the functional group 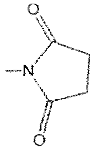 in the compound A and compound B to the functional group -NH₂ in the oligopeptide is 1:(1 to 2).

8. The sealing hydrogel according to claim 1, wherein the first component further contains a color developer, the ratio of the color developer to the total mass of compound A and compound B in the first component is (0.0001 to 0.01):1.

9. The sealing hydrogel according to claim 1, wherein the number of residues of amino acids in the oligopeptide is from 2 to 6.

10. The sealing hydrogel according to claim 1 or 8, wherein the second component further comprises a bacteriostatic agent, the bacteriostatic agent is a chitosan derivative, the mass ratio of the bacteriostatic agent to the oligopeptide is (0.015 to 0.1):1.

11. The sealing hydrogel according to claim 1, wherein the first component and/or the second component is added with a drug, the drug is at least one of an anti-inflammatory drug, a hemostatic drug, an analgesic drug, an anti-rejection drug and an anti-tumor drug.

12. The sealing hydrogel according to any one of claims 1 to 11, wherein the sealing hydrogel is prepared by the following method:
(1) mixing the first component and the second component with buffer a to obtain a mixed solution system; and
(2) mixing buffer b with the mixed solution system to obtain a sealing hydrogel; the buffer a has a pH of 6.0 to 7.5; and/or, the buffer b has a pH of 9.0 to 9.5; in particular
wherein in the mixed solution system, the oligopeptide has a concentration of 1 to 7 mg/mL;
and/or, in the mixed solution system, the total concentration of compound A and compound B is 40 to 100 mg/mL; more particularly
wherein the buffer a contains one or more of phosphate, sodium chloride, potassium chloride, phosphoric acid and hydrochloric acid; and/or,
the buffer b contains one or more of phosphate, carbonate, borate and sodium hydroxide; most particularly
wherein a volume ratio of the buffer a to the buffer b is 1:(0.8 to 1.2).

13. A method for preparing the sealing hydrogel according to any one of claims 1 to 12, **characterized by** comprising the following steps:
(1) mixing the first component and the second component with buffer a to obtain a mixed solution system; and
(2) mixing buffer b with the mixed solution system to obtain a sealing hydrogel; the buffer a has a pH of 6.0 to 7.5; and/or, the buffer b has a pH of 9.0 to 9.5.

14. A kit for preparing the sealing hydrogel of any one of claims 1 to 12, wherein, a duplex syringe is arranged in the kit, the first component and second component are dissolved in buffer a and stored in syringe 1; and the buffer b is stored in syringe 2.

15. A sealing hydrogel of any one of claims 1 to 12 for use in the sealing of spinal dura mater and cerebral dura mater, leak sealing of other tissues, wound sealing and anti-adhesion.

## Patentansprüche

1. Versiegelungshydrogel, wobei Vorläufer zur Herstellung des versiegelnden Hydrogels eine erste Komponente und eine zweite Komponente umfassen;
die erste Komponente umfasst Verbindung A und Verbindung B; die Verbindung A ist ein mehrarmiges Polyethylenglycolderivat, und die Verbindung B ist ein mehrarmiges Copolyester aus Glycidylisopropylether und Ethylglycidylether;
die zweite Komponente ein Oligopeptid umfasst.

2. Versiegelungshydrogel nach Anspruch 1, wobei sowohl die Verbindung A als auch die Verbindung B die folgende Gruppe enthalten:

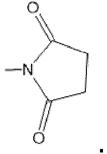

3. Versiegelungshydrogel nach Anspruch 1 oder 2, wobei die Verbindung A die allgemeine Strukturformel hat: 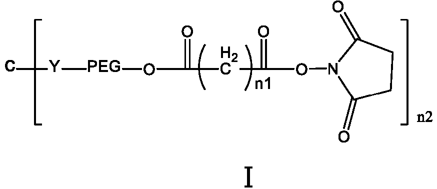
wobei n1 eine ganze Zahl von 2 bis 8 und n2 eine ganze Zahl von 4 bis 8 ist; das Molekulargewicht von PEG 1250 bis 8000 beträgt und der PDI von PEG ≤ 1,02 ist;
Y eine gleiche oder unterschiedliche Verbindungsgruppe ist, die aus einer oder mehrerer der folgenden Gruppen ausgewählt ist: -O-, -O(CH₂CH₂)ᵢ-, -(CH₂CH₂)ᵢ-, - (CH₂)ᵢCONH-, -OOC(CH₂)ᵢCOO-, -OOCNH(CH₂)ᵢNHCOO- and -OOC(CH₂)ᵢCONH-; und i eine ganze Zahl von 0 bis 20 ist;
die Verbindung B die allgemeine Strukturformel hat: 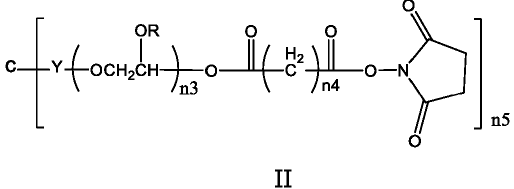
wobei n3 eine ganze Zahl von 11 bis 70 ist, n4 eine ganze Zahl von 2 bis 8 ist und n5 eine ganze Zahl von 4 bis 8 ist;
die Gruppen, aus denen Y in der allgemeinen Formel II ausgewählt werden kann, sind dieselben wie die Gruppen, aus denen Y in der allgemeinen Formel I ausgewählt werden kann; und R ist Ethyl oder Isopropyl.

4. Versiegelungshydrogel nach einem der Ansprüche 1 bis 3, wobei das versiegelnde Hydrogel ein Schwellverhältnis in dreidimensionaler Richtung von größer oder gleich -5 % und kleiner oder gleich 5% aufweist.

5. Versiegelungshydrogel nach Anspruch 1, wobei in der Verbindung B pro 100 Teile Ethylglycidylether und Glycidylysopropylether 60 bis 80 Teile Ethylglycidylether umfasst sind.

6. Versiegelungshydrogel nach Anspruch 1, wobei die Verbindung A und die Verbindung B die gleiche Anzahl an Armen aufweisen; oder
wobei die Verbindung A und die Verbindung B das gleiche Molekulargewicht aufweisen; oder
wobei in 100 Teilen der Mischung aus Verbindung A und Verbindung B 40 bis 60 Teile Verbindung A umfasst sind.

7. Versiegelungshydrogel nach einem der Ansprüche 2 bis 6, wobei das Molverhältnis der funktionellen Gruppe 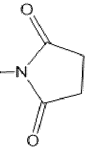 in der Verbindung A und der Verbindung B zu der funktionellen Gruppe -NH₂ im Oligopeptid 1:(1 bis 2) beträgt.

8. Versiegelungshydrogel nach Anspruch 1, wobei die erste Komponente ferner einen Farbentwickler enthält, wobei das Verhältnis des Farbentwicklers zur Gesamtmasse der Verbindung A und der Verbindung B in der ersten Komponente (0,0001 bis 0,01):1 beträgt.

9. Versiegelungshydrogel nach Anspruch 1, wobei die Anzahl der Aminosäurereste im Oligopeptid 2 bis 6 beträgt.

10. Versiegelungshydrogel nach Anspruch 1 oder 8, wobei die zweite Komponente zusätzlich ein bakteriostatisches Mittel umfasst, das bakteriostatische Mittel ein Chitosan-Derivat ist und das Massenverhältnis des bakteriostatischen Mittels zu dem Oligopeptid (0,015 bis 0,1):1 beträgt.

11. Versiegelungshydrogel nach Anspruch 1, wobei der ersten Komponente und/oder der zweiten Komponente ein Arzneimittel zugesetzt ist, wobei das Arzneimittel mindestens eines von einem entzündungshemmenden Arzneimittel, einem blutstillenden Arzneimittel, einem schmerzstillenden Arzneimittel, einem abstossungshemmenden Arzneimittel und einem tumorhemmenden Arzneimittel ist.

12. Versiegelungshydrogel nach einem der Ansprüche 1 bis 11, wobei das Versiegelungshydrogel durch das folgende Verfahren hergestellt wird:
(1) Mischen der ersten Komponente und der zweiten Komponente mit Puffer a, um ein gemischtes Lösungssystem zu erhalten; und
(2) Mischen des Puffers b mit dem gemischten Lösungssystem, um ein Versiegelungshydrogel zu erhalten; wobei der Puffer a einen pH-Wert von 6,0 bis 7,5 aufweist; und/oder der Puffer b einen pH-Wert von 9,0 bis 9,5 aufweist; insbesondere
wobei in dem gemischten Lösungssystem das Oligopeptid eine Konzentration von 1 bis 7 mg/ml aufweist;
und/oder in dem gemischten Lösungssystem die Gesamtkonzentration von Verbindung A und Verbindung B 40 bis 100 mg/ml beträgt; insbesondere
wobei der Puffer a eines oder mehrere der folgenden enthält: Phosphat, Natriumchlorid, Kaliumchlorid, Phosphorsäure und Salzsäure; und/oder
der Puffer b eines oder mehrere der folgenden enthält: Phosphat, Carbonat, Borat und Natriumhydroxid; insbesondere
wobei das Volumenverhältnis von Puffer a zu Puffer b 1:(0,8 bis 1,2) beträgt.

13. Verfahren zur Herstellung des Versiegelungshydrogel gemäß einem der Ansprüche 1 bis 12, **gekennzeichnet durch** die folgenden Schritte:
(1) Mischen der ersten Komponente und der zweiten Komponente mit Puffer a, um ein gemischtes Lösungssystem zu erhalten; und
(2) Mischen des Puffers b mit dem gemischten Lösungssystem, um ein Versiegelungshydrogel zu erhalten; wobei der Puffer a einen pH-Wert von 6,0 bis 7,5 aufweist und/oder der Puffer b einen pH-Wert von 9,0 bis 9,5 aufweist.

14. Kit zur Herstellung des Versiegelungshydrogel nach einem der Ansprüche 1 bis 12, wobei in dem Kit eine Doppelspritze angeordnet ist, die erste Komponente und die zweite Komponente in Puffer a gelöst und in Spritze 1 gelagert sind; und der Puffer b in Spritze 2 gelagert ist.

15. Versiegelungshydrogel nach einem der Ansprüche 1 bis 12 zur Verwendung zum Versiegeln der spinalen Dura mater und der zerebralen Dura mater, zum Versiegeln von anderen Geweben, zum Versiegeln von Wunden und zur Verhinderung von Adhäsionen.

## Revendications

1. Hydrogel d'étanchéité, dans lequel des précurseurs pour la préparation de l'hydrogel d'étanchéité comprennent un premier composant et un deuxième composant;
le premier composant comprend un composé A et un composé B; le composé A est un dérivé de polyéthylène glycol à plusieurs bras, et le composé B est un copolyester à plusieurs bras de glycidyl isopropyl éther et d'éthyl glycidyl éther;
le deuxième composant comprend un oligopeptide.

2. Hydrogel d'étanchéité selon la revendication 1, dans lequel le composé A et le composé B contiennent tous deux le groupe suivant:

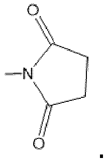

3. Hydrogel d'étanchéité selon la revendication 1 ou 2, dans lequel le composé A a la formule structurale générale suivante : 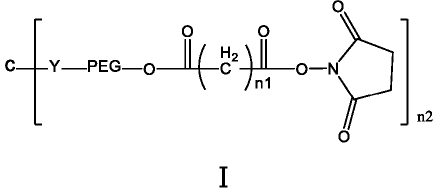
dans laquelle n1 est un nombre entier compris entre 2 et 8 et n2 est un nombre entier compris entre 4 et 8; le poids moléculaire du PEG est compris entre 1250 et 8000, et le PDI du PEG est ≤ 1,02;
Y est un groupe de liaison identique ou différent qui est un ou plusieurs choisi parmi -O-, -O(CH₂CH₂)ᵢ-, -(CH₂CH₂)ᵢ-, -(CH₂)ᵢCONH-, -OOC(CH₂)ᵢCOO-, -OOCNH(CH₂)ᵢNHCOO- et -OOC(CH₂)ᵢCONH-; et i est un nombre entier compris entre 0 et 20;
le composé B a une formule structurale générale: 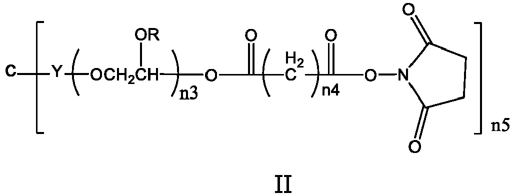
dans laquelle n3 est un nombre entier compris entre 11 et 70, n4 est un nombre entier compris entre 2 et 8, et n5 est un nombre entier compris entre 4 et 8; les groupes dont Y dans la formule générale II peut être choisi sont les mêmes que ceux dont Y dans la formule générale I peut être choisi; et R est un groupe éthyle ou isopropyle.

4. Hydrogel d'étanchéité selon l'une quelconque des revendications 1 à 3, dans lequel l'hydrogel d'étanchéité a un rapport de gonflement dans la direction tridimensionnelle supérieur ou égal à -5 % et inférieur ou égal à 5 %.

5. Hydrogel d'étanchéité selon la revendication 1, dans lequel, dans le composé B, pour 100 parties d'éther éthylique de glycidyle et d'éther isopropylique de glycidyle, il y a 60 à 80 parties d'éther éthylique de glycidyle.

6. Hydrogel d'étanchéité selon la revendication 1, dans lequel le composé A et le composé B ont le même nombre de bras; ou
dans lequel le composé A et le composé B ont le même poids moléculaire; ou
dans lequel, pour 100 parties du mélange du composé A et du composé B, il y a 40 à 60 parties du composé A.

7. Hydrogel d'étanchéité selon l'une quelconque des revendications 2 à 6, dans lequel le rapport molaire entre le groupe fonctionnel 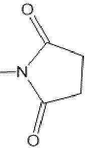 dans le composé A et le composé B et le groupe fonctionnel -NH₂ dans l'oligopeptide est de 1:(1 à 2).

8. Hydrogel d'étanchéité selon la revendication 1, dans lequel le premier composant contient en outre un révélateur de couleur, le rapport entre le révélateur de couleur et la masse totale du composé A et du composé B dans le premier composant est de (0,0001 et 0,01):1.

9. Hydrogel d'étanchéité selon la revendication 1, dans lequel le nombre de résidus d'acides aminés dans l'oligopeptide est compris entre 2 et 6.

10. Hydrogel d'étanchéité selon la revendication 1 ou 8, dans lequel le deuxième composant comprend en outre un agent bactériostatique, l'agent bactériostatique est un dérivé de chitosane, le rapport massique de l'agent bactériostatique à l'oligopeptide est de (0,015 à 0,1):1.

11. Hydrogel d'étanchéité selon la revendication 1, dans lequel le premier composant et/ou le deuxième composant est additionné avec un médicament, le médicament étant au moins l'un parmi un médicament anti-inflammatoire, un médicament hémostatique, un médicament analgésique, un médicament anti-rejet et un médicament anti-tumoral.

12. Hydrogel d'étanchéité selon l'une quelconque des revendications 1 à 11, dans lequel l'hydrogel d'étanchéité est préparé par le procédé suivant:
(1) mélanger du premier composant et du deuxième composant avec un tampon a pour obtenir un système de solution mélangée; et
(2) mélanger le tampon b avec le système de solution mélangée pour obtenir un hydrogel d'étanchéité; le tampon a a un pH de 6,0 à 7,5; et/ou le tampon b a un pH de 9,0 à 9,5; en particulier
dans lequel, dans le système de solution mélangée, l'oligopeptide a une concentration de 1 à 7 mg/mL;
et/ou, dans le système de solution mélangée, la concentration totale du composé A et du composé B est comprise entre 40 et 100 mg/mL; plus particulièrement
dans lequel le tampon a contient un ou plusieurs de phosphate, chlorure de sodium, chlorure de potassium, acide phosphorique et acide chlorhydrique; et/ou,
le tampon b contient un ou plusieurs de phosphate, carbonate, borate et hydroxyde de sodium; plus particulièrement
dans lequel le rapport volumique du tampon a au tampon b est de 1:(0,8 à 1,2).

13. Procédé de préparation de l'hydrogel d'étanchéité selon l'une quelconque des revendications 1 à 12, **caractérisé en ce qu'**il comprend les étapes suivantes:
(1) mélanger le premier composant et le deuxième composant avec le tampon a pour obtenir un système de solution mélangée; et
(2) mélanger le tampon b avec le système de solution mélangée pour obtenir un hydrogel d'étanchéité; le tampon a a un pH de 6,0 à 7,5; et/ou le tampon b a un pH de 9,0 à 9,5.

14. Kit pour préparer l'hydrogel d'étanchéité selon l'une quelconque des revendications 1 à 12, dans lequel une seringue double est disposée dans le kit, le premier composant et le deuxième composant sont dissous dans le tampon a et stockés dans la seringue 1; et le tampon b est stocké dans la seringue 2.

15. Hydrogel d'étanchéité selon l'une quelconque des revendications 1 à 12, destiné à être utilisé pour étanchéifier la dure-mère spinale et la dure-mère cérébrale, pour étanchéiser des fuites dans d'autres tissus, pour étanchéiser des plaies et pour empêcher l'adhérence.
